# EUROPÄISCHE PATENTANMELDUNG

(11) **EP 0 856 289 A1**
(43) Veröffentlichungstag der Anmeldung: **05.08.1998**
(21) Anmeldenummer: 98101572.0
(22) Anmeldetag: 29.01.1998
(51) Int. Cl.: A61B 17/22, A61D 1/02

(54) **Stenosestanze**

(30) Priorität: 31.01.1997 DE 19703698
(71) Anmelder: Medl, Susanne, Dr., 87727 Babenhausen (DE)
(72) Erfinder: Medl, Maximilian, Dr., verstorben (DE)
(74) Vertreter: DIEHL GLAESER HILTL & PARTNER

(57) **Zusammenfassung**

Angegeben wird eine Stenosestanze zur Behandlung von Strichkanalstenosen, insbesondere bei Milchkühen, mit einer Schneidvorrichtung, die eine hohlzylindrische, an ihrem Oberende geschlossene Messerscheide mit einer seitlichen Schneidöffnung und ein in der Messerscheide axial verschiebbares und drehbares hohlzylindrisches Rundmesser mit einer Handhabe aufweist. Die Stanze ist dadurch gekennzeichnet, daß der Unterabschnitt der Messerscheide (1) als Griffteil (3) mit Längsschlitzen (14, 15) und einem Umfangsschlitz ausgebildet ist, die Handhabe (26) an einer Stelle der Schlitze (14, 15) aus diesen herausragt und in diesen verschiebbar ist und daß verschiedene Stellungen der Handhabe (26) bestimmten Stellungen von Schneidkanten des Rundmessers (17) entsprechen. Die Stenosestanze gestattet Schnitte in verschiedene Richtungen.

## Beschreibung

Die Erfindung betrifft eine Stenosestanze zur Behandlung von Strichkanalstenosen, insbesondere bei Milchkühen.

Bei Kühen tritt des öfteren das Problem der Schwermelkbarkeit auf. Dabei kann die Milch nur unter einem unerwünscht hohen Kraft- und Zeitaufwand von der Milchkuh abgenommen werden. Die Milchabflußstörungen können den Milchfluß nicht nur verzögern, sondern sogar teilweise oder ganz unterbrechen.

Die Milchabflußstörungen können an jedem Ort des milchabführenden Systems auftreten, das einen Teil des Euters der Milchkuh bildet. Das Euter ist ein mächtiger Drüsenkörper, der aus jeweils zwei Vordervierteln und zwei Hintervierteln besteht. Jedes Viertel ist eine vollständig von den anderen Vierteln abgeschlossene Milchdrüse mit eigener Zitze. Ein solches Viertel gliedert sich in ein milchbildendes Drüsengewebe und ein milchabführendes System. Letzteres besteht aus den Milchgängchen, den Milchgängen, der Drüsenzisterne, der Zitzenzisterne und dem Strichkanal.

Die Ursachen einer Schwermelkbarkeit können Verletzungen, Quetschungen und Entzündungen des Euters, insbesondere der Zitze, sein. In etwa 95 % der Fälle liegt die Ursache der Schwermelkbarkeit im Strichkanal. Seine Länge beträgt etwa 8 bis 14 mm.

Im Strichkanalbereich muß die medizinisch konservative, gegebenenfalls chirurgische Behandlung unter größtmöglicher Schonung des Gewebes im Strichkanal erfolgen, weil jede dieser Behandlungsarten eine Infektionsgefahr und damit das Risiko einer Entzündung des Euters mit sich bringt.

Es sind verschiedene medizinische Methoden und Vorrichtungen zur Behandlung der durch Zitzenstörungen verursachten Schwermelkbarkeit oder der sogenannten Zitzenstenose bekannt. Unter anderem ist in der Veröffentlichung der internationalen Patentanmeldung WO 95/22943 eine Gewebsresektorzange zur Behandlung von Strichkanalstenosen beschrieben. Diese Zange besteht im wesentlichen aus einem Griffteil mit zwei gegeneinander verschwenkbaren Griffschenkeln, einem die Griffschenkel in ihre Öffnungsrichtung drückenden Federelement und einer mittels der Griffschenkel betätigbaren Schneidvorrichtung. Letztere weist eine hohlzylindrische, an ihrem Oberende geschlossene Messerscheide mit einer seitlichen Schneidöffnung zur Aufnahme von zu entfernendem Gewebe am Ort der Stenose und ein in der Messerscheide sowohl axial verschiebbares als auch um seine Längsachse drehbares, im wesentlichen hohlzylindrisches Rundmesser mit einer Handhabe auf. Bei dieser Schneidvorrichtung muß der Schneidvorgang im Strichkanal immer von unten nach oben erfolgen, d. h. die Schneidkante am Oberende des Rundmessers muß sich dabei in Richtung auf das Einführende der Zange bewegen.

Stenosen sind aber häufig ungleichmäßig ausgebildet. Für Gewebsresektionen bedeutet dies, daß die optimale Schneidrichtung vom Einzelfall abhängt. Manchmal ist es zweckmäßig, im Strichkanal den Schnitt in das Gewebe von unten nach oben zu führen. In anderen Fällen kann ein Schnitt von oben nach unten besser sein, und in wiederum anderen Fällen ist ein Schnitt von der Seite sinnvoll. Die Möglichkeit, eine Gewebsresektorzange wahlweise für mindestens zwei verschiedene Schneidrichtungen einzusetzen, ist aber bei der bekannten Gewebsresektorzange oder auch anderen bereits beschriebenen Schneidvorrichtungen zur Behandlung von Strichkanalstenosen nicht gegeben.

Die Stenosen befinden sich häufig an der sogenannten Fürstenbergschen Rosette, die sich (vom Innern der Milchdrüse aus gesehen) am Strichkanaleingang befindet. Es wäre deshalb für den Operateur besonders hilfreich, wenn er sein chirurgisches Schneidinstrument an dem stenosierenden Gewebe einfach und schnell positionieren könnte, obwohl er es von außen nicht sehen kann.

Der Erfindung liegt daher einerseits die Aufgabe zugrunde, eine Stenosestanze zur Behandlung von Strichkanalstenosen, insbesondere bei Milchkühen, anzugeben, die es ermöglicht, im Strichkanal durch Schnitte in verschiedenen Richtungen Gewebeteile zu entfernen. Auch soll die Stenosestanze so ausgebildet sein, daß bei ihrem Einführen in einen Strichkanal oder ihrem Herausziehen aus diesem das Verletzungsrisiko durch scharfe Kanten an der Stenosestanze möglichst vermieden wird und ein sicheres Entfernen der abgeschnittenen Gewebeteile aus dem Strichkanal gewährleistet ist. Eine zusätzliche Aufgabe besteht darin, eine Stenosestanze anzugeben, die einfach und schnell im Strichkanal richtig positioniert werden kann, um Schnitte an der Fürstenbergschen Rosette durchzuführen.

Diese Aufgaben löst die vorliegende Erfindung gemäß einer ersten Ausführungsform durch eine Stenosestanze, wie sie im Patentanspruch 1 definiert ist, mit einer Schneidvorrichtung, die eine hohlzylindrische, an ihrem Oberende geschlossene Messerscheide mit einer seitlichen Schneidöffnung zur Aufnahme von zu entfernendem Strichkanalgewebe und ein in der Messerscheide sowohl axial verschiebbares als auch um seine Längsachse drehbares, im wesentlichen hohlzylindrisches Rundmesser mit einer Handhabe aufweist. Die Stenosestanze ist dadurch gekennzeichnet, daß der Unterabschnitt der Messerscheide als Griffteil der Stenosestanze ausgebildet ist, der mit zwei unterschiedlich langen Längsschlitzen und einem diese Längsschlitze verbindenden Umfangsschlitz versehen ist, daß die Handhabe des Rundmessers an irgendeiner Stelle innerhalb der Schlitze aus diesen herausragt und in diesen verschiebbar ist und daß verschiedene Stellungen dieser Handhabe innerhalb der Schlitze bestimmten Stellungen von Schneidkanten des Rundmessers an der Schneidöffnung der Messerscheide entsprechen.

Bei einer zweiten Ausführungsform der Erfindung handelt es sich um eine Stenosestanze, wie sie im Patentanspruch 8 definiert ist, mit einer Schneidvorrichtung, die eine hohlzylindrische, an ihrem Oberende geschlossene Messerscheide mit einer seitlichen Schneidöffnung zur Aufnahme von zu entfernendem Strichkanalgewebe und ein in der Messerscheide sowohl axial verschiebbares als auch um seine Längsachse drehbares, im wesentlichen hohlzylindrisches Rundmesser mit einer Handhabe aufweist. Diese Stenosestanze ist dadurch gekennzeichnet, daß der Unterabschnitt der Messerscheide als Griffteil der Stenosestanze ausgebildet ist, der mit einem Längsschlitz und einem Umfangsschlitz, der sich an das obere Ende des Längsschlitzes anschließt, versehen ist, daß die Handhabe des Rundmessers an irgendeiner Stelle innerhalb der Schlitze aus diesen herausragt und in diesen verschiebbar ist, daß verschiedene Stellungen dieser Handhabe innerhalb der Schlitze bestimmten Stellungen von Schneidkanten des Rundmessers an der Schneidöffnung der Messerscheide entsprechen und daß ein Einführteil in einem Längsabschnitt unterhalb der Schneidöffnung verjüngt ist und dadurch eine Umfangsschulter am Einführteil ausgebildet ist.

Die erste Ausführungsform der erfindungsgemäßen Stenosestanze hat den wesentlichen Vorteil, daß sie drei Schneidvorrichtungen in einer einzigen Vorrichtung vereinigt. Das heißt, mit der Stenosestanze können in einem Strichkanal Schnitte wahlweise von unten nach oben, von oben nach unten und von der Seite durchgeführt werden. Dabei können auch ohne Veränderung der Position der Stenosestanze innerhalb eines Strichkanals die verschiedenen Schnittrichtungen unmittelbar nacheinander und in beliebiger Reihenfolge praktiziert werden.

Die Stenosestanze hat den weiteren Vorteil, daß ihre Schneidöffnung jederzeit sowohl innerhalb als auch außerhalb des Strichkanals geschlossen werden kann, d. h. bei Bedarf das Freiliegen irgendeiner der Schneidkanten des Rundmessers vermieden wird. Dies beseitigt das Risiko unerwünschter Verletzungen durch freie Schneidkanten, sowohl beim Operateur als auch bei dem zu behandelnden Tier. Die Gefahr einer solchen Verletzung des Tieres ist beispielsweise dann gegeben, wenn das Tier beim Einführen der Stenosestanze in den Strichkanal oder beim Entfernen hieraus unruhig wird.

Das Schließen der Schneidöffnung hat ferner den Vorteil, daß die im Strichkanal abgeschnittenen Gewebeteile, die sich innerhalb des Rundmessers befinden, beim Entnehmen der Stenosestanze aus dem Strichkanal nicht ungewollt abgestreift werden können, sondern sicher mit herausgeholt werden.

Wesentliche Merkmale der zweiten Ausführungsform der Erfindung sind die Verjüngung des Einführteils unterhalb der Schneidöffnung und die Umfangsschulter am oberen Beginn dieser Verjüngung. Diese Ausführungsform ist insbesondere zum Entfernen von stenosierendem Gewebe an der vorgenannten Fürstenbergschen Rosette vorgesehen. Die erwähnte Verjüngung und die Umfangsschulter des Einführteils der Stenosestanze erleichtern sehr das richtige Positionieren der Stenosestanze an der Rosette.

Die Patentansprüche 2 bis 7 betreffen bevorzugte Merkmale der ersten Ausführungsform. Die Patentansprüche 9 und 10 beziehen sich auf bevorzugte Merkmale der zweiten Ausführungsform der Erfindung. Die Patentansprüche 11 bis 20 sind auf bevorzugte Merkmale sowohl der ersten als auch der zweiten Ausführungsform gerichtet.

Im Zusammenhang mit der vorliegenden Erfindung beziehen sich die Ausdrücke "oben, unten, Oberende, Unterende, oberer Anschlag, unterer Anschlag" und ähnliche Formulierungen auf die Position der Stenosestanze bei ihrer Benutzung, d. h. auf die Position, bei der die Stenosestanze senkrecht angeordnet ist und mit ihrem Einführende und ihrer Schneidöffnung von unten nach oben in einen Strichkanal eingeführt worden ist. Dementsprechend ist das Einführende der Stenosestanze auch deren Oberende und das auf der entgegengesetzten Seite liegende Griffende der Stenosestanze deren Unterende.

Nachfolgend wird die Erfindung durch in der Zeichnung schematisch dargestellte Ausführungsbeispiele erläutert. Die Fig. 1 bis 5d beziehen sich auf die erste, die Fig. 6 bis 8a auf die zweite Ausführungsform.

Es zeigen:
- Fig. 1: eine Seitenansicht einer ersten Ausführungsform einer Messerscheide der Stenosestanze, teilweise im Schnitt, wobei das Rundmesser mit zugehöriger Handhabe und Druckfeder weggelassen sind sowie die Verschlußkappe am Unterende des Griffteils in abgeschraubtem Zustand dargestellt ist;
- Fig. 2: einen Längsschnitt eines Rundmessers, das für den Einsatz in die Messerscheide gemäß Fig. 1 geeignet ist, mit der zugehörigen Druckfeder am Unterende sowie einer abgeschraubten ersten Handhabe mit Nippel und einer alternativ verwendbaren abgeschraubten zweiten Handhabe ohne Nippel;
- Fig.3a,3b und 3c: Seitenansichten, teilweise im Schnitt, der aus der Messerscheide gemäß Fig. 1 und dem Rundmesser gemäß Fig. 2 zusammengesetzten Stenosestanze mit drei verschiedenen Positionen des Rundmessers für einen Schnitt von unten nach oben;
- Fig.3d,3e und 3f: vergrößerte Seitenansichten, teilweise im Schnitt, des Bereichs der Schneidöffnung der Stenosestanze gemäß Fig. 3a, 3b und 3c, mit drei verschiedenen Positionen des Rundmessers für einen Schnitt von unten nach oben;
- Fig.4a und 4b: Seitenansichten, teilweise im Schnitt, der aus der Messerscheide gemäß Fig. 1 und dem Rundmesser gemäß Fig. 2 zusammengesetzten Stenosestanze mit zwei verschiedenen Positionen des Rundmessers für einen Schnitt von der Seite;
- Fig.4c und 4d: vergrößerte Seitenansichten, teilweise im Schnitt, des Bereichs der Schneidöffnung der Stenosestanze gemäß Fig. 4a und 4b, mit zwei verschiedenen Positionen des Rundmessers für einen Schnitt von der Seite;
- Fig.5a und 5b: Seitenansichten, teilweise im Schnitt, der aus der Messerscheide gemäß Fig. 1 und dem Rundmesser gemäß Fig. 2 zusammengesetzten Stenosestanze mit zwei verschiedenen Positionen des Rundmessers für einen Schnitt von oben nach unten;
- Fig.5c und 5d: vergrößerte Seitenansichten, teilweise im Schnitt, des Bereichs der Schneidöffnung der Stenosestanze gemäß Fig. 5a und 5b, mit zwei verschiedenen Positionen des Rundmessers für einen Schnitt von oben nach unten;
- Fig. 6: einen vergrößerten Längsschnitt des oberen Abschnitts der Messerscheide einer zweiten Ausführungsform;
- Fig. 7: einen vergrößerten Längsschnitt des oberen Abschnitts eines Rundmessers, das für den Einsatz in die Messerscheide gemäß Fig. 6 geeignet ist;
- Fig.8a: einen vergrößerten Längsschnitt des oberen Abschnitts des Einführteils der Stenosestanze, die aus der Messerscheide gemäß Fig. 6 und dem Rundmesser gemäß Fig. 7 zusammengesetzt ist und sich in der Position vor dem Schneiden von oben nach unten befindet; und
- Fig.8b: einen Längsschnitt entsprechend Fig. 8a, wobei sich aber die Stenosestanze in der Position nach dem Schneiden von oben nach unten befindet.

Nachfolgend wird zunächst die erste Ausführungsform der erfindungsgemäßen Stenosestanze beschrieben.

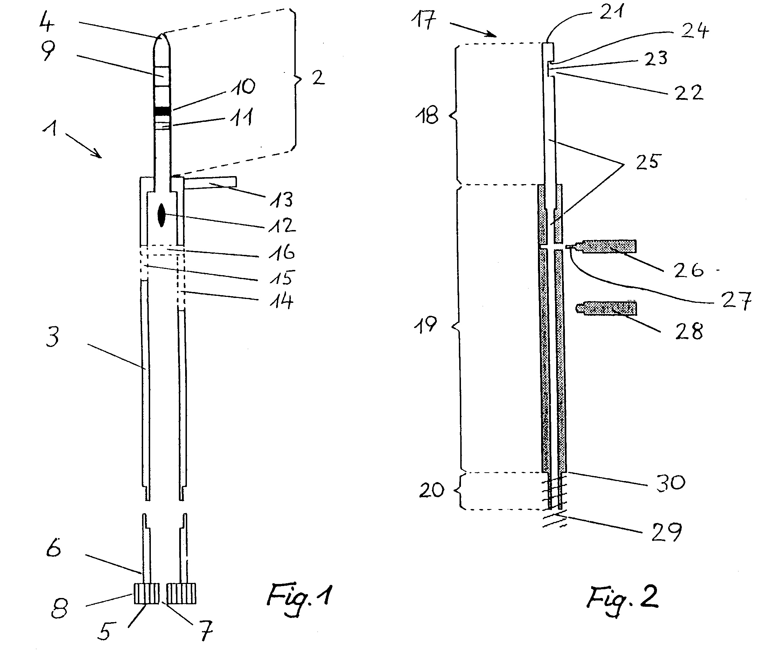

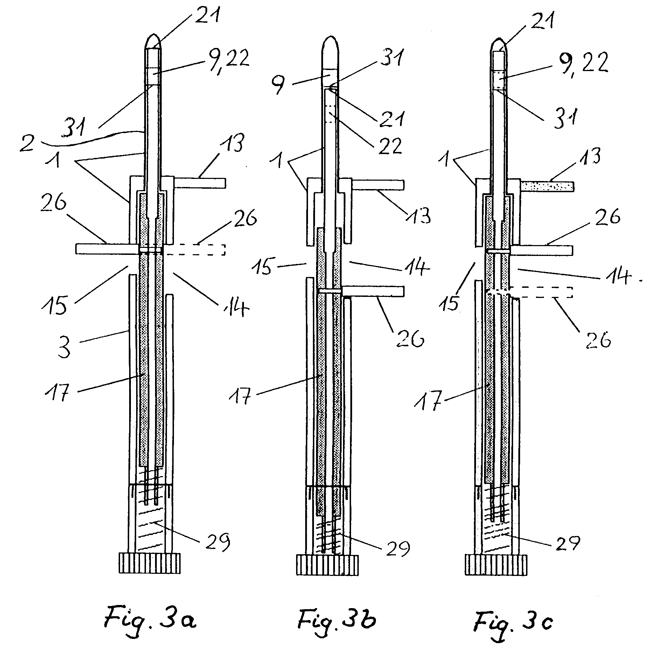

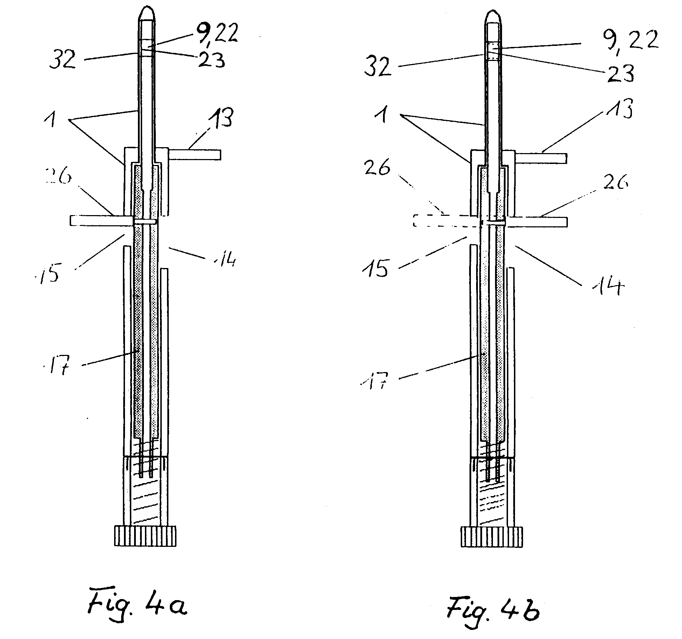

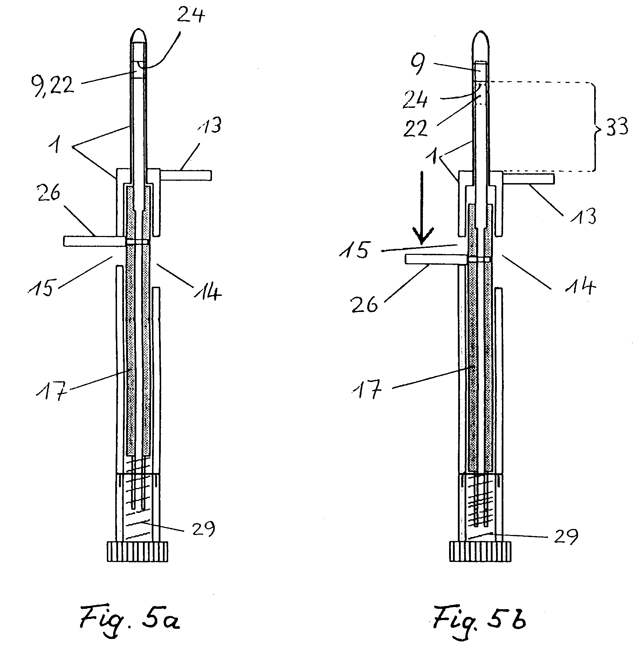

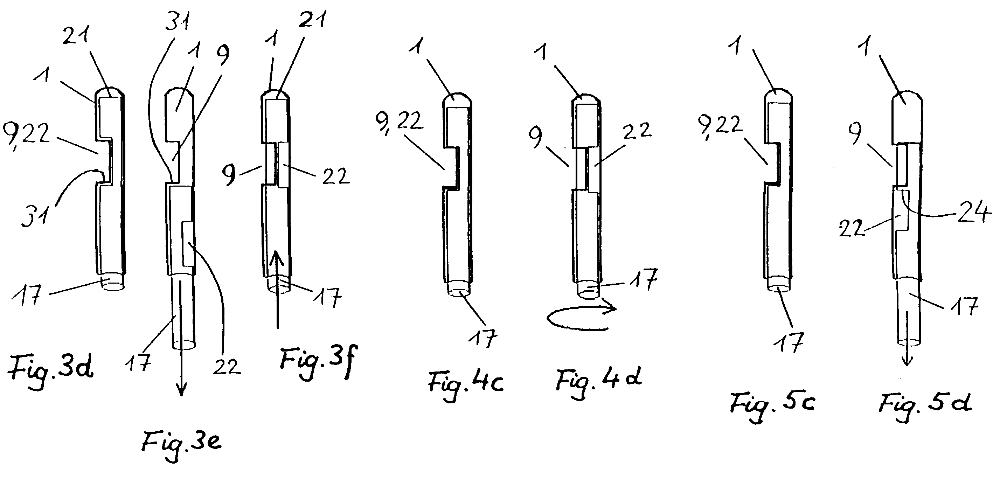

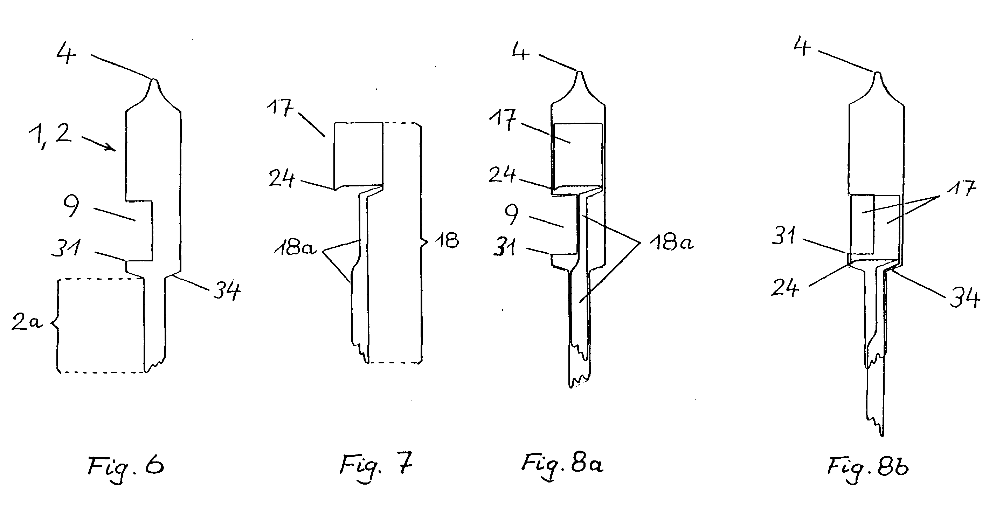

In den Fig. 1 und 2 sind die Teile der Stenosestanze in zerlegter Form dargestellt. In der zusammengesetzten Form ist die Stenosestanze jeweils aus den Fig. 3a, 3b und 3 c oder den Fig. 4a und 4b oder den Fig. 5a und 5b ersichtlich.

In Fig. 1 ist eine hohlzylindrische Messerscheide 1 dargestellt. Sie weist in ihrem oberen Bereich einen Einführteil 2 mit einem relativ kleinen Durchmesser und in ihrem unteren Bereich einen Griffteil 3 mit einem im Vergleich zum Einführteil 2 größeren Durchmesser auf. Die Messerscheide 1 ist an ihrem Oberende oder Einführende 4 geschlossen. Am gegenüberliegenden Unterende oder Griffende 5 ist die Messerscheide 1 durch eine abschraubbare Kappe 6 verschlossen, die in Fig. 1 in abgeschraubtem Zustand dargestellt ist. Die Kappe 6 ist mit einer zentralen Bohrung 7 versehen und mit einer Rändelschraube 8 zum leichteren Drehen von Hand ausgerüstet.

Im Einführteil 2 sind eine seitliche Schneidöffnung 9 und darunter Farbmarkierungen 10, 11 ausgebildet. Die Schneidöffnung 9 ist etwa 8 bis 9 mm vom Einführende 4 entfernt. Die Farbmarkierungen 10, 11 dienen dem Operateur zur Abschätzung der Eindringtiefe des Einführteils 2 in einen Strichkanal.

Der Griffteil 3 trägt in seinem oberen Bereich unterhalb der Schneidöffnung 9 eine Markierung 12, welche dem Operateur die Position der Schneidöffnung 9 am Umfang des Einführteils 2 anzeigt, wenn die Stenosestanze in einen Strichkanal eingeführt ist. Ferner befindet sich im oberen Bereich des Griffteils 3 ein daran befestigter, seitlich vorragender Haltearm 13. Schließlich ist der Griffteil 3 mit zwei unterschiedlich langen Längsschlitzen 14, 15 und einem diese Längsschlitze verbindenden Umfangsschlitz 16 versehen.

Im zusammengesetzten Zustand der Stenosestanze, wie er schematisch aus den Fig. 3a bis 3c, 4a, 4b, 5a und 5b ersichtlich ist, befindet sich ein darin sowohl axial verschiebbares als auch um seine Längsachse drehbares, im wesentlichen hohlzylindrisches Rundmesser 17. Gemäß Fig. 2 besteht das Rundmesser 17 aus einem oberen Abschnitt 18 mit einem relativ kleinen Durchmesser, einem mittleren Abschnitt 19 mit einem relativ großen Durchmesser und einem unteren Abschnitt 20 mit einem relativ kleinen Durchmesser.

Bei der zusammengesetzten Stenosestanze befindet sich der obere Abschnitt 18 des Rundmessers 17 größtenteils im Einführteil 2 der Messerscheide 1, während der mittlere Abschnitt 19 und der untere Abschnitt 20 des Rundmessers 17 im Griffteil 3 angeordnet sind.

Das Rundmesser 17 weist an seinem Oberende eine nach oben gerichtete erste Schneidkante 21 für ein Schneiden von unten nach oben sowie an einer seitlichen Messeröffnung 22 eine seitwärts gerichtete zweite Schneidkante 23 für ein Schneiden in Umfangsrichtung des Rundmessers 17 und am Oberende der seitlichen Messeröffnung 22 eine nach unten gerichtete dritte Schneidkante für ein Schneiden von oben nach unten auf.

In dem Rundmesser 17 ist ein durchgehender Hohlraum 25 vorgesehen, der sich vom Oberende, an dem sich die Schneidkanten 21, 23, 24 befinden, bis zum Unterende des unteren Abschnitts 20 erstreckt. Im mittleren Abschnitt 19 des Rundmessers 17 ist eine Handhabe 26 radial eingeschraubt. In Fig. 2 ist diese Handhabe 26 im herausgeschraubten Zustand dargestellt. Die Handhabe 26 ist an ihrem Einschraubende mit einem Nippel 27 versehen. Wenn die Handhabe 26 in das Rundmesser 17 eingeschraubt ist, unterbricht der Nippel 27 den Durchgang des Hohlraums 25. Dies ist beispielsweise aus Fig. 3a bis 3c, 4a, 4b, 5a und 5b ersichtlich. Alternativ kann anstelle der Handhabe 26 mit dem Nippel 27 auch eine Handhabe 28 ohne diesen Nippel verwendet werden, mit der der Durchgang des Hohlraums 25 nicht unterbrochen wird. Welche der Handhaben 26, 28 benutzt wird, hängt davon ab, ob an der Stenosestanze ein freier Durchgang von der Schneidöffnung 9 in der Messerscheide 1 bis zur Bohrung 7 in der Kappe 6 des Griffteils 3 erwünscht ist, zum Beispiel für den Durchtritt von Milch aus dem Strichkanal, in den die Stenosestanze eingesetzt ist, oder ob ein solcher Durchgang nicht bestehen soll.

Bei zusammengesetzter Stenosestanze ragt die Handhabe 26, 28 des Rundmessers 17 an irgendeiner Stelle innerhalb der Schlitze 14, 15, 16 aus dem Griffteil 3 heraus und ist in den Schlitzen 14, 15, 16 verschiebbar. Da mit der Verschiebung der Handhabe 26, 28 auch das Rundmesser 17 in der Messerscheide 1 verschoben wird, entsprechen verschiedene Stellungen der Handhabe 26, 28 bestimmten Stellungen der Schneidkanten 21, 23, 24 des Rundmessers 17 an der Schneidöffnung 9 der Messerscheide 1. Mit anderen Worten, die Schlitze 14, 15, 16 stellen in ihrer Gesamtheit eine Art Kulisse dar, in der die Handhabe 26, 28 bei ihrer Verschiebung geführt wird.

Aus Fig. 1 ist ersichtlich, daß sich der lange Längsschlitz 14 und der kurze Längsschlitz 15 im Griffteil 3 von beiden Enden des Umfangsschlitzes 16 aus in Richtung zum Unterende 5 des Griffteils 3 erstrecken. Die Längsschlitze 14, 15 weisen voneinander einen Winkelabstand von etwa 180° in Umfangsrichtung des Griffteils 3 auf. Der lange Längsschlitz 14 ist etwa doppelt so lang wie der kurze Längsschlitz 15.

Das Rundmesser 17 wird mittels einer Druckfeder 29 in Richtung auf eine Stellung gedrückt, in der sich die Handhabe 26, 28 des Rundmessers 17 am oberen Anschlag eines der beiden Längsschlitze 14, 15 oder im Umfangsschlitz 16 des Griffteils 3 befindet. Die Druckfeder 29 ist eine Schraubenfeder, die einerseits an der am Unterende des Griffteils 3 aufgeschraubten Kappe 6 und andererseits an einer Umfangsschulter 30 des Rundmessers abgestützt ist, wie beispielsweise in den Fig. 3a bis 3c, 4a, 4b, 5a und 5b ersichtlich ist.

In der axialen Draufsicht auf den Einführteil 2 der Stenosestanze beträgt der Winkelabstand zwischen der Handhabe 26, 28 des Rundmessers 17 und dem Haltearm 13 des Griffteils 3 weniger als 180°, vorzugsweise etwa 145°. Dadurch ist eine gute Handhabbarkeit der Stenosestanze sichergestellt.

Nachfolgend werden die beim Gebrauch der Stenosestanze möglichen Stellungen der Handhabe 26, 28 und des damit geführten Rundmessers 17 innerhalb der Messerscheide 1 und die entsprechend davon abhängigen Stellungen der Schneidkanten 21, 23, 24 an der Schneidöffnung 9 der Messerscheide 1 erläutert. Aus dieser Erläuterung geht gleichzeitig die Benutzung der Stenosestanze hervor.

Die Fig. 3a, 3b, 3c, 3d, 3e und 3f beziehen sich auf einen Schneidvorgang von unten nach oben mittels der ersten Schneidkante 21 des Rundmessers 17. Die Fig. 3a, 3b und 3c erläutern Stellungen des Rundmessers 17 in der Messerscheide 1 und die Fig. 3d, 3e und 3f die jeweils zugehörigen Stellungen der Messeröffnung 22 des Rundmessers 17 an der Schneidöffnung 9 der Messerscheide 1. Bei den Fig. 3a, 3b und 3c blickt der Betrachter in die Schneidöffnung 9 und bei der Fig. 3a zusätzlich in die Messeröffnung 22. Die gestrichelt gezeichnete Messeröffnung 22 in Fig. 3b und 3 c bedeutet, daß sie sich auf der vom Betrachter abgewandten Seite der Stenosestanze befindet. Die Darstellung der Stenosestanze in den Fig. 3d, 3e und 3f ist gegenüber jener in den Fig. 3a, 3b und 3c um 90° nach links gedreht.

In der Ausgangsstellung des Rundmessers 17 gemäß Fig. 3a befindet sich die Handhabe 26 am oberen Anschlag des kurzen Längsschlitzes 15 des Griffteils 3. Dieser Stellung entspricht eine Stellung der Messeröffnung 22 des Rundmessers 17 gemäß Fig. 3d, d.h. die letztere ist deckungsgleich mit der Schneidöffnung 9 der Messerscheide 1 angeordnet. Um einen Schneidvorgang von unten nach oben zu ermöglichen, wird die Handhabe 26 im Umfangsschlitz 16 (Fig. 1) horizontal nach rechts in die obere Anschlagstellung des langen Längsschlitzes 14 geschwenkt (gestrichelte Handhabe 26 in Fig. 3a) und dann entgegen der Wirkung der Druckfeder 29 in diesem Schlitz 14 bis zu dessen unterem Anschlag gezogen. Diese Stellung ist in Fig. 3b dargestellt. Ihr entspricht die Stellung der Messeröffnung 22 gemäß Fig. 3e, worin die Schneidöffnung 9 der Messerscheide 1 nach links und die weiter unten befindliche Messeröffnung 22 des Rundmessers 17 nach rechts gerichtet ist. Außerdem ist dabei die erste Schneidkante 21 am Unterende 31 der Schneidöffnung 9 der Messerscheide 1 angeordnet, und die Schneidöffnung 9 ist offen. In dieser Stellung der Stenosestanze im Strichkanal wird der abzuschneidende Gewebeteil durch entsprechenden Druck des Operateurs auf die Außenseite der Zitze in die Schneidöffnung 9 hineingedrückt, worauf dann der Operateur die nach unten gezogene Handhabe 26 entlastet. Dadurch wird das Rundmesser 17 durch die Wirkung der Druckfeder 29 wieder zum oberen Anschlag des langen Längsschlitzes 14 bewegt, wobei gleichzeitig der in der Schneidöffnung 9 befindliche Gewebeteil durch die erste Schneidkante 21 in der Schneidöffnung 9 abgeschnitten und vom Hohlraum 25 (Fig. 2) des Rundmessers 17 aufgenommen wird. Die dann erreichte Endstellung des Rundmessers ist in Fig. 3c dargestellt (die gestrichelte Handhabe 26 entspricht der Stellung gemäß Fig. 3b). Die entsprechende Stellung der Messeröffnung 22 ist aus Fig. 3f ersichtlich. Diese zeigt insbesondere, daß die Messeröffnung 22 des Rundmessers 17 von der Schneidöffnung 9 der Messerscheide 1 abgewandt ist, d. h. diese Schneidöffnung 9 durch das Rundmesser 17 verschlossen wird. In dieser Stellung ist beim Einführen der Stenosestanze in einen Strichkanal oder beim Herausziehen aus diesem ein Verletzungsrisiko ausgeschlossen.

Die Fig. 4a, 4b, 4c und 4d beziehen sich auf einen seitlichen, d. h. in Umfangsrichtung des Rundmessers 17 gerichteten Schneidvorgang mittels der seitwärts gerichteten zweiten Schneidkante 23 des Rundmessers 17. In der Darstellung gemäß den Fig. 4a und 4b bewegt sich dabei die zweite Schneidkante 23 in der Schneidöffnung 9 von links nach rechts. Für diesen Schneidvorgang erläutern die Fig. 4a und 4b Stellungen des Rundmessers 17 in der Messerscheide 1 und die Fig. 4c und 4d die jeweils zugehörigen Stellungen der Messeröffnung 22 des Rundmessers 17 an der Schneidöffnung 9 der Messerscheide 1. Bei den Fig. 4a und 4b blickt der Betrachter in die Schneidöffnung 9 und bei der Fig. 4a zusätzlich in die Messeröffnung 22. Die gestrichelt gezeichnete Messeröffnung 22 in Fig. 4b bedeutet, daß sie sich auf der vom Betrachter abgewandten Seite der Stenosestanze befindet. Die Darstellung der Stenosestanze in den Fig. 4c und 4d ist gegenüber jener in den Fig. 4a und 4b um 90° nach links gedreht.

In der Ausgangsstellung des Rundmessers 17 gemäß Fig. 4a, die gleich der Stellung gemäß Fig. 3a ist, befindet sich die Handhabe 26 am oberen Anschlag des kurzen Längsschlitzes 15 des Griffteils 3. Dieser Stellung entspricht eine Stellung der Messeröffnung 22 des Rundmessers 17 gemäß Fig. 4c, die mit der Fig. 3d übereinstimmt. Das heißt, die zweite Schneidkante 23 ist an einem Seitenrand 32 der Schneidöffnung 9 der Messerscheide 1 angeordnet. Die Messeröffnung 22 des Rundmessers 17 ist dabei deckungsgleich mit der Schneidöffnung 9 der Messerscheide 1. Wenn sich die Stenosestanze im Strichkanal befindet, wird in der Stellung gemäß Fig. 4c der abzuschneidende Gewebeteil durch entsprechenden Druck des Operateurs auf die Zitzenaußenseite in die Schneidöffnung 9 eingedrückt. Anschließend wird die Handhabe 26 im Umfangsschlitz 16 (Fig. 1) zum oberen Anschlag des langen Längsschlitzes 14 geschwenkt, wobei sich die Stellung des Rundmessers 17 gemäß Fig. 4b ergibt (die gestrichelte Handhabe 26 zeigt die Ausgangsstellung). Die Schwenkbewegung wird durch den Pfeil in Fig. 4d symbolisiert, wo auch die dann erreichte Endstellung der Messeröffnung 22 gezeigt wird, welche jener gemäß Fig. 3f entspricht. Das heißt, die Messeröffnung 22 des Rundmessers 17 weist in die entgegengesetzte Richtung zur Richtung der Schneidöffnung 9 der Messerscheide 1. Dadurch ist diese Schneidöffnung 9 durch das Rundmesser 17 verschlossen, wodurch eine weitere Verletzung des Strichkanals vermieden wird. Während des Verschwenkens der Handhabe 26 in die Stellung gemäß Fig. 4d wird der in die Schneidöffnung eingedrückte Gewebeteil mittels der zweiten Schneidkante 23 von der Seite abgeschnitten und in den Hohlraum 25 des Rundmessers 17 aufgenommen.

In der Stellung gemäß Fig. 4b ist die erste Schneidkante 21 des Rundmessers 17 in einen Längsabschnitt 1a der Messerscheide 1 oberhalb ihrer Schneidöffnung 9 und die zweite Schneidkante 23 in den Umfangsabschnitt der Messerscheide 1 neben der Schneidöffnung 9 eingeschoben.

Die Fig. 5a, 5b, 5c und 5d beziehen sich auf einen Schneidvorgang des Rundmessers 17 von oben nach unten mittels der dritten Schneidkante 24 am Oberende der Messeröffnung 22 des Rundmessers 17. Die Fig. 5a und 5b erläutern Stellungen des Rundmessers 17 in der Messerscheide 1 und die Fig. 5c und 5d die jeweils zugehörigen Stellungen der Messeröffnung 22 des Rundmessers 17 an der Schneidöffnung 9 der Messerscheide 1. Bei den Fig. 5a und 5b blickt der Betrachter in die Schneidöffnung 9 und bei der Fig. 5a zusätzlich in die Messeröffnung 22. Die gestrichelt gezeichnete Messeröffnung 22 in Fig. 5b bedeutet, daß sie sich auf der vom Betrachter abgewandten Seite der Stenosestanze befindet. Die Darstellung der Stenosestanze in den Fig. 5c und 5d ist gegenüber jener in den Fig. 5a und 5b um 90° nach links gedreht.

Die Ausgangsstellung des Rundmessers 17 gemäß Fig. 5a entspricht den Ausgangsstellungen gemäß Fig. 3a und 4a. Das heißt, die Handhabe 26 befindet sich im oberen Anschlag des kurzen Längsschlitzes 15 des Griffteils 3. Dieser Stellung entspricht eine Stellung der Messeröffnung 22 des Rundmessers 17 gemäß Fig. 5c, d.h. die letztere ist deckungsgleich mit der Schneidöffnung 9 der Messerscheide 1 angeordnet. Wenn sich die Stenosestanze in diesem Zustand in einem zu behandelnden Strichkanal befindet, wird der abzuschneidende Gewebeteil in die Schneidöffnung 9 eingedrückt. Dann drückt der Operateur die Handhabe 26 entsprechend dem Pfeil in Fig. 5b nach unten bis zum unteren Anschlag des kurzen Längsschlitzes 15. Dies entspricht einer Abwärtsbewegung der Messeröffnung 22 gemäß dem Pfeil in Fig. 5d, wobei die dritte Schneidkante 24 des Rundmessers 17 über die Schneidöffnung 9 geführt wird und gleichzeitig den darin eingedrückten Gewebeteil abschneidet. Dieser wird vom Hohlraum 25 (Fig. 2) aufgenommen. In der Stellung gemäß Fig. 5b ist die dritte Schneidkante 24 des Rundmessers 17 in einen Längsabschnitt 33 des Einführteils 2 (Fig. 1) der Messerscheide 1 unterhalb der Schneidöffnung 9 eingeschoben. Dabei ist die Schneidöffnung 9 durch das Rundmesser 17 verschlossen. Somit besteht keine Gefahr, beim Herausziehen der Stenosestanze aus dem Strichkanal das Gewebe weiter zu verletzen. Nach dem Entfernen der Stenosestanze aus dem Strichkanal entlastet der Operateur wieder die Handhabe 26, und das Rundmesser 17 kehrt durch die Wirkung der Druckfeder 29 in die Ausgangsstellung gemäß Fig. 5a zurück.

Nach jeder Benutzung der Stenosestanze kann diese leicht gereinigt und insbesondere von dem im Hohlraum 25 des Rundmessers 17 vorliegenden abgeschnittenen Gewebeteil befreit werden. Dazu wird am Griffteil 3 der Messerscheide 1 die Kappe 6 mittels der Rändelschraube 8 abgeschraubt (Fig. 1). Ferner wird die Handhabe 26, 28 aus dem Rundmesser 17 herausgeschraubt. Dann ist es möglich, das Rundmesser 17 zusammen mit der Druckfeder 29 aus der Messerscheide 1 nach unten herauszuziehen und entsprechend zu reinigen. Nach der Reinigung erfolgt das Zusammensetzen der Stenosestanze in umgekehrter Reihenfolge.

Die zweite Ausführungsform der erfindungsgemäßen Stenosestanze, die in den Fig. 6, 7, 8a und 8b erläutert ist, entspricht weitgehend der ersten Ausführungsform. Ein Unterschied besteht darin, daß bei der zweiten Ausführungsform der Einführteil 2 in einem Längsschnitt 2a unterhalb der Schneidöffnung 9 verjüngt ist und dadurch am Beginn der Verjüngung an dem Einführteil 2 eine Verjüngungsschulter 34 ausgebildet ist (Fig. 6). Letztere liegt nur wenig unterhalb des Unterendes 31 der Schneidöffnung 9. Deshalb ist die zweite Ausführungsform der erfindungsgemäßen Stenosestanze nicht für Schnitte von unten nach oben vorgesehen, sondern für Schnitte von oben nach unten und gegebenenfalls zusätzlich für Schnitte von der Seite, wie sie für die erste Ausführungsform beschrieben wurden.

In Fig. 7 ist der obere Abschnitt 18 des Rundmessers 17 mit der Schneidkante 24 für das Schneiden von oben nach unten dargestellt.

Fig. 8a zeigt die Teile gemäß Fig. 6 und Fig. 7 in der zusammengesetzten Form in der Ruheposition der Stenosestanze. Aus dieser Darstellung wird auch deutlich, daß das Rundmesser 17 in einem Abschnitt 18a unterhalb der Schneidkante 24 entsprechend dem verjüngten Längsabschnitt 2a der Messerscheide 1 gleichfalls verjüngt ist, damit es beim Schneiden von oben nach unten in der Messerscheide 1 so weit nach unten gezogen werden kann, daß die Schneidkante 24 noch etwas unterhalb des Unterendes 31 der Schneidöffnung 9 gelangt. Diese Position nach dem Schneidvorgang ist aus Fig. 8b ersichtlich.

Bei der zweiten Ausführungsform gemäß Fig. 6, 7, 8a und 8b ist die Verjüngung an der Schulter 34 konzentrisch ausgebildet. Die Verjüngung kann aber in beliebiger Weise exzentrisch gestaltet sein. Die Form des Rundmessers 17 ist dann dieser Gestalt angepaßt.

Bei der Benutzung der Stenosestanze gemäß der zweiten Ausführungsform wird beim Schneiden von oben nach unten oder, falls das Rundmesser 17 zusätzlich zur nach unten gerichteten Schneidkante 24 eine zur Seite gerichtete Schneidkante 23 aufweist, beim Schneiden von der Seite wie bei der ersten Ausführungsform vorgegangen.

Um stenosierendes Gewebe an der Fürstenbergschen Rosette zu entfernen, verfährt man bei der zweiten Ausführungsform wie folgt:

Die durch den Strichkanal eingeführte Stenosestanze wird bis in die Zitzenzisterne vorgeschoben, bis die Schneidöffnung 9 den Strichkanal wieder verläßt. Anschließend wird die Stanze wieder zurückgezogen. Dabei tritt ein fühlbarer Widerstand auf, wenn die Verjüngungsschulter 34 den Strichkanaleingang, d. h. die Fürstenbergsche Rosette, erreicht. Durch geringfügiges weiteres Zurückziehen der Stenosestanze gelangt das Gewebe der Rosette in den Bereich der Schneidöffnung 9, insbesondere an deren Unterkante 31. Damit ist die richtige Position der Stanze zur Resektion von stenosierendem Gewebe an der Rosette erreicht und der Schnitt kann mit der Schneidkante 24 von oben nach unten oder mit der Schneidkante 23 von der Seite erfolgen. Die Verjüngung am Längsabschnitt 2a des Einführteils 2, zusammen mit der Verjüngungsschulter 34, erleichtern also wesentlich das richtige Positionieren der Stenosestanze an der Rosette.

## Patentansprüche

1. Stenosestanze zur Behandlung von Strichkanalstenosen, insbesondere bei Milchkühen, mit einer Schneidvorrichtung, die eine hohlzylindrische, an ihrem Oberende geschlossene Messerscheide mit einer seitlichen Schneidöffnung zur Aufnahme von zu entfernendem Strichkanalgewebe und ein in der Messerscheide sowohl axial verschiebbares als auch um seine Längsachse drehbares, im wesentlichen hohlzylindrisches Rundmesser mit einer Handhabe aufweist, dadurch gekennzeichnet, daß der Unterabschnitt der Messerscheide (1) als Griffteil (3) der Stenosestanze ausgebildet ist, der mit zwei unterschiedlich langen Längsschlitzen (14, 15) und einem diese Längsschlitze (14, 15) verbindenden Umfangsschlitz (16) versehen ist, daß die Handhabe (26, 28) des Rundmessers (17) an irgendeiner Stelle innerhalb der Schlitze (14, 15, 16) aus diesen herausragt und in diesen verschiebbar ist und daß verschiedene Stellungen dieser Handhabe (26, 28) innerhalb der Schlitze (14, 15, 16) bestimmten Stellungen von verschiedenen Schneidkanten (21, 23, 24) des Rundmessers (17) an der Schneidöffnung (9) der Messerscheide (1) entsprechen.

2. Stenosestanze nach Anspruch 1, dadurch gekennzeichnet, daß sich die beiden Längsschlitze (14, 15) im Griffteil (3) von den Enden des Umfangsschlitzes (16) aus in Richtung zum Unterende (5) des Griffteils (3) erstrecken.

3. Stenosestanze nach Anspruch 1 oder 2, dadurch gekennzeichnet, daß die Längsschlitze (14, 15) im Griffteil (3) voneinander einen Winkelabstand von etwa 180° in Umfangsrichtung des Griffteils (3) aufweisen.

4. Stenosestanze nach einem der Ansprüche 1 bis 3, dadurch gekennzeichnet, daß der erste Längsschlitz (14) im Griffteil (3) etwa doppelt so lang ist wie der zweite Längsschlitz (15).

5. Stenosestanze nach einem der Ansprüche 1 bis 4, dadurch gekennzeichnet, daß das Rundmesser (17) an seinem Oberende eine nach oben gerichtete erste Schneidkante (21) für ein Schneiden von unten nach oben sowie an einer seitlichen Messeröffnung (22) eine seitwärts gerichtete zweite Schneidkante (23) für ein Schneiden in Umfangsrichtung des Rundmessers (17) und am Oberende der seitlichen Messeröffnung (22) eine nach unten gerichtete dritte Schneidkante (24) für ein Schneiden von oben nach unten aufweist.

6. Stenosestanze nach einem der Ansprüche 1 bis 5, dadurch gekennzeichnet, daß dann, wenn sich die Handhabe (26, 28) des Rundmessers (17) am unteren Anschlag des langen Längsschlitzes (14) des Griffteils (3) befindet, die erste Schneidkante (21) des Rundmessers (17) am Unterende (31) der Schneidöffnung (9) der Messerscheide (1) angeordnet ist, wobei die Schneidöffnung (9) offen ist.

7. Stenosestanze nach einem der Ansprüche 1 bis 5, dadurch gekennzeichnet, daß dann, wenn sich die Handhabe (26, 28) des Rundmessers (17) am oberen Anschlag des langen Längsschlitzes (14) befindet, die erste Schneidkante (21) des Rundmessers (17) hinter den Längsabschnitt der Messerscheide (1) oberhalb ihrer Schneidöffnung (9) und die zweite Schneidkante (23) des Rundmessers (17) hinter den Umfangsabschnitt der Messerscheide (1) neben der Schneidöffnung (9) eingeschoben sind, wobei das Rundmesser (17) diese Schneidöffnung (9) verschließt.

8. Stenosestanze zur Behandlung von Strichkanalstenosen, insbesondere bei Milchkühen, mit einer Schneidvorrichtung, die eine hohlzylindrische, an ihrem Oberende geschlossene Messerscheide mit einer seitlichen Schneidöffnung zur Aufnahme von zu entfernendem Strichkanalgewebe und ein in der Messerscheide sowohl axial verschiebbares als auch um seine Längsachse drehbares, im wesentlichen hohlzylindrisches Rundmesser mit einer Handhabe aufweist, dadurch gekennzeichnet, daß der Unterabschnitt der Messerscheide (1) als Griffteil (3) der Stenosestanze ausgebildet ist, der mit einem Längsschlitz (15) und einem Umfangsschlitz (16), der sich an das obere Ende des Längsschlitzes (15) anschließt, versehen ist, daß die Handhabe (26, 28) des Rundmessers (17) an irgendeiner Stelle innerhalb der Schlitze (15, 16) aus diesen herausragt und in diesen verschiebbar ist, daß verschiedene Stellungen dieser Handhabe (26, 28) innerhalb der Schlitze (15, 16) bestimmten Stellungen von verschiedenen Schneidkanten (23, 24) des Rundmessers (17) an der Schneidöffnung (9) der Messerscheide (1) entsprechen und daß ein Einführteil (2) in einem Längsabschnitt (2a) unterhalb der Schneidöffnung (9) verjüngt ist und dadurch eine Verjüngungsschulter (34) am Einführteil (2) ausgebildet ist.

9. Stenosestanze nach Anspruch 8, dadurch gekennzeichnet, daß das Rundmesser (17) am Oberende der seitlichen Messeröffnung (22) eine nach unten gerichtete Schneidkante (24) für ein Schneiden von oben nach unten und gegebenenfalls zusätzlich an der seitlichen Messeröffnung (22) eine seitwärts gerichtete Schneidkante (23) für ein Schneiden in Umfangsrichtung des Rundmessers (17) aufweist.

10. Stenosestanze nach Anspruch 8 oder 9, dadurch gekennzeichnet, daß dann, wenn sich die Handhabe (26, 28) des Rundmessers (17) am oberen Anschlag des Längsschlitzes (14) befindet, die Schneidkante (23) für ein Schneiden in Umfangsrichtung des Rundmessers (17) hinter den Umfangs-abschnitt der Messerscheide (1) neben der Schneidöffnung (9) eingeschoben ist, wobei das Rundmesser (17) diese Schneidöffnung (9) verschließt.

11. Stenosestanze nach einem der Ansprüche 1 bis 10, dadurch gekennzeichnet, daß das Rundmesser (17) mittels einer Druckfeder (29) in Richtung auf eine Stellung gedrückt wird, in der sich die Handhabe (26, 28) des Rundmessers (17) am oberen Anschlag eines Längsschlitzes (14, 15) oder im Umfangsschlitz (16) des Griffteils (3) befindet.

12. Stenosestanze nach Anspruch 11, dadurch gekennzeichnet, daß die Druckfeder (29) eine Spiralfeder ist, die einerseits an einer am Unterende (5) des Griffteils (3) aufgeschraubten Kappe (6) und andererseits an einer Umfangsschulter (30) des Rundmessers (17) abgestützt ist.

13. Stenosestanze nach einem der Ansprüche 1 bis 12, dadurch gekennzeichnet, daß dann, wenn sich die Handhabe (26, 28) des Rundmessers (17) am oberen Anschlag des Längsschlitzes (15) des Griffteils (3) befindet, die Schneidkante (23) für ein Schneiden in Umfangsrichtung des Rundmessers (17) an einem Seitenrand (32) der Schneidöffnung (9) der Messerscheide (1) und die Schneidkante (24) für ein Schneiden von oben nach unten am Oberende dieser Schneidöffnung (9) angeordnet ist, wobei diese Schneidöffnung (9) offen ist.

14. Stenosestanze nach einem der Ansprüche 1 bis 12, dadurch gekennzeichnet, daß dann, wenn sich die Handhabe (26, 28) des Rundmessers (17) am unteren Anschlag des Längsschlitzes (15) des Griffteils (3) befindet, die Schneidkante (24) des Rundmessers (17) für ein Schneiden von oben nach unten hinter den Längsabschnitt (33) des Einführteils (2) der Messerscheide (1) unterhalb der Schneidöffnung (9) eingeschoben ist, wobei das Rundmesser (17) diese Schneidöffnung (9) verschließt.

15. Stenosestanze nach einem der Ansprüche 1 bis 14, dadurch gekennzeichnet, daß am Oberende des Griffteils (3) ein seitlich vorragender Haltearm (13) befestigt ist.

16. Stenosestanze nach Anspruch 15, dadurch gekennzeichnet, daß in der axialen Draufsicht auf den Einführteil (2) der Stenosestanze der Winkelabstand zwischen der Handhabe (26, 28) des Rundmessers (17) und dem Haltearm (13) des Griffteils (3) weniger als 180° beträgt.

17. Stenosestanze nach einem der Ansprüche 1 bis 16, dadurch gekennzeichnet, daß das Rundmesser (17) von seinem Oberende bis zu seinem Unterende einen durchgehenden Hohlraum (25) und das Unterende (5) des Griffteils (3) eine diesen Hohlraum (25) nach außen öffnende Bohrung (7) aufweist.

18. Stenosestanze nach Anspruch 17, dadurch gekennzeichnet, daß die Handhabe (26, 28) in das Rundmesser (17) lösbar eingeschraubt ist und an ihrem Einschraubende wahlweise ohne (28) oder mit (26) einem vorspringenden Nippel (27) versehen ist, der im Falle seines Vorhandenseins bei eingeschraubter Handhabe (26) den Durchgang durch den Hohlraum (25) des Rundmessers (17) unterbricht.

19. Stenosestanze nach einem der Ansprüche 1 bis 18, dadurch gekennzeichnet, daß unterhalb der Schneidöffnung (9) am Griffteil (3) eine Markierung (12) angebracht ist.

20. Stenosestanze nach einem der Ansprüche 1 bis 19, dadurch gekennzeichnet, daß am Einführteil (2) der Messerscheide (1) unterhalb der Schneidöffnung (9) in mindestens einem Abstand vom Einführende (4) der Stenosestanze eine Markierung (10, 11) angebracht ist.
